Europäisches Patentamt

European Patent Office

Office européen des brevets

(11) Publication number: **0 414 688 B1**

(12) **EUROPEAN PATENT SPECIFICATION**

(45) Date of publication of patent specification: **24.08.94**

(51) Int. Cl.⁵: **A61K 31/19**, A61K 31/60, A61K 33/06, A61K 33/08, A61K 47/02

(21) Application number: **89902387.3**

(22) Date of filing: **15.02.89**

(86) International application number:
**PCT/FI89/00024**

(87) International publication number:
**WO 89/07439 (24.08.89 89/20)**

The file contains technical information submitted after the application was filed and not included in this specification

(54) **PROCESS FOR REGULATING THE ABSORPTION RATE OF DRUGS AND A PHARMACEUTICAL FORMULATION.**

(30) Priority: **16.02.88 FI 880712**

(43) Date of publication of application:
**06.03.91 Bulletin 91/10**

(45) Publication of the grant of the patent:
**24.08.94 Bulletin 94/34**

(84) Designated Contracting States:
**AT BE CH DE FR GB IT LI LU NL SE**

(56) References cited:
**EP-A- 0 083 775**
**CA-A- 1 234 761**
**US-A- 4 217 340**
**US-A- 4 294 819**

**D'Arcy & Mc Elnay in Drug Intelligence & Clinical pharmacy 21, 607-617, 1987**

(73) Proprietor: **NEUVONEN, Pertti J.**
**Halkosuonatie 81-83 G**
**SF-00660 Helsinki (FI)**

(72) Inventor: **NEUVONEN, Pertti J.**
**Halkosuonatie 81-83 G**
**SF-00660 Helsinki (FI)**

(74) Representative: **Bergvall, Stina-Lena et al**
**Dr. Ludwig Brann Patentbyra AB**
**P.O. Box 17192**
**S-104 62 Stockholm (SE)**

Chemical Abstracts, vol. 110, no. 13, March 13, 1989, P.J. Neuvonen et al.: "Effect of magnesiumhydroxid on the absorption of tolfenamic and mefenamic acids", abstract number 87908 p

Chemical Abstracts, vol. 83, 1975, M. Nozaki et al: "Effects of combined use of non-steroidal antiinflammatory drugs and ant-acids", abstract number 71750

Dialog Informational Services, file 155, Med-line 66-89, accesion number 04460031, Tobert J.A. et al. "Effect of antacids on the bioavailability of diflunisal in the fasting and postprandial states".

EP 0 414 688 B1

## Description

The present invention relates to a process and its applications for preparing pharmaceutical formulations having an appropriate, i.e. accelerated or retarded rate of absorption.

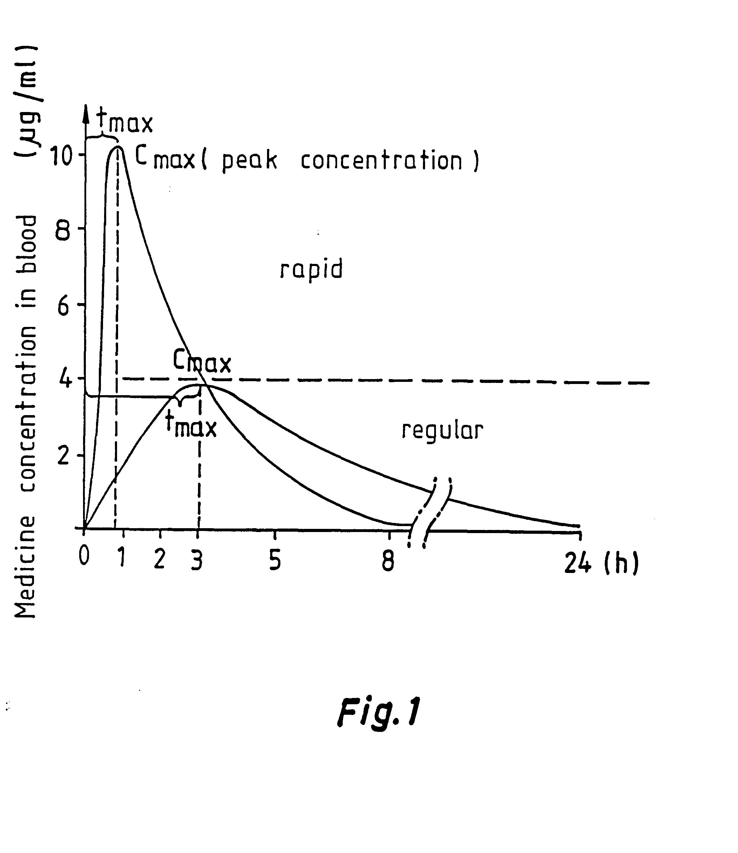

In oral administration of medicines, a slow absorption rate of the drug is frequently a problem when a rapid and anticipated medical effect is required. If absorption is too slow, the elimination of a drug occurring simultaneously with the absorption period may prevent the concentrations from rising to a required therapeutic level even if the total absorption of a drug were complete (fig. 1). This problem is particularly pronounced when using drugs with a short half-life e.g. for sudden attacks of pain. For example, a budding pain of migraine or gout may have time to develop as a result of slow drug absorption to the extent that an orally administered usual dosage of medicine takes a poor effect or no effect at all. This is why drugs must often be administered intramuscularly or even intravenously for assuring a rapid absorption rate. This way of administering is not only inconvenient and requires medical expertise but also there are no such injectable forms available of nearly all drugs.

The absorption of tolfenamic acid from alimentary canal is relatively slow (PJ Pentikäinen, PJ Neuvonen & C Backman: European Journal of Clinical Pharmacology 1984:17:67-75) and, thus, a therapeutical concentration and desired medical effect cannot often be attained.

In fact, intense pain generally slows down emptying of the stomach and, thus, slows down also the absorption of medicines. By means of a rapidly absorbing formulation it would also be possible to reduce the need and use of injection-administered medicines (painkillers).

On the other hand, a slower-than-normal absorption of medicines (e.g. fenamates) would be necessary a.o. in the treatment of chronic inflammatory conditions, e.g. rheumatic diseases. A slow rate of absorption would prolong the duration of medical effect and facilitate fewer dosages. This would be significant especially with medicines having a short half-life, since a slow absorption rate would then serve as a factor limiting the reduction of medicine concentrations in plasma (so-called flip-flop phenomenon). At the same time, a slow absorption rate would restrain the development of too high medicine concentrations causing side effects and, thus, it would be possible to increase single dosages.

A slow absorption rate is a problem when using fenamate class medicines e.g. for migraine patients but the same problem applies to many other drugs as well. In case of fenamates, attempts have been made to solve this problem e.g. by using a micronized raw material, by administering methoclopramide along with fenamates for quicker emptying of the stomach, by taking caffeine along with fenamates or by making medical formulations in the form of suppositories, but the results have been poor. The inadequate effect of a medicine is often a result of the fact that the absorption rate is too slow even if the total absorption were complete.

US-A-4 217 340 (Merck & Co., Inc.) relates to a rapid acting drug combination comprising a phenyl bensoic acid compound and magnesium hydroxide.

Nozaki et al., Proc. Symp. Drug Metab. Action, 5th 1973, 105-15, 1974 describes compositions comprising NASAIDs and antacids and shows that antacids increase NSAIDs' absorption. Table 2, page 109 discloses a composition comprising mefenamic acid and MgO.

Garnham et al., Postgraduate Medical Journal, vol. 53, 126-129, 1977 describes compositions comprising indomethacin and either sodium bicarbonate or aluminium hydroxide and studies the effect of these two antacides on the absorption rate of indomethacin.

The present invention, however, relates to a formulation with a regulated absorption rate comprising a carboxylic acid derivative selected from tolfenamic acid and ibuprofen and an alkaline compound selected from $Mg(OH)_2$, MgO and a mixture of these compounds

By means of this invention it is possible to regulate, to accelerate or retard the absorption of drugs from alimentary canal into blood circulation. The most important advantage is that the top concentration of medicine in blood can be attained at any given instant by accelerating or retarding the absorption as necessary. On the basis of the invention it is possible to optimize an absorption profile which is significant as a safety-improving factor e.g. by eliminating the administration of unnecessarily large doses of medicine. By virtue of accelerated absorption it is possible to attain a temporarily high medicine concentration even with a regular dose of medicine, which is desirable and appropriate in certain clinical situations.

A process of the invention is characterized in that the agent for regulating the rate of absorption comprises $Mg(OH)_2$ or MgO or a mixture thereof.

The drug is an orally administered medicine having an absorption profile which is too slow or too rapid in view of its intended application, such as
- tolfenamic acid and
  ibuprofen,

3

A drug of the invention having a regulated absorption rate contains, in addition to conventional formulation components, as an absorption rate regulating agent some alkaline compound, such as $Mg(OH)_2$, MgO, or a mixture thereof.

Preferred compounds in a process of the invention comprise acidic antiinflammatory painkillers such as tolfenamic acid or its salts or propionic acids, such as ibuprofen or its salts.

The invention will now be described in more detail with reference made to the accompanying drawings and the following examples.

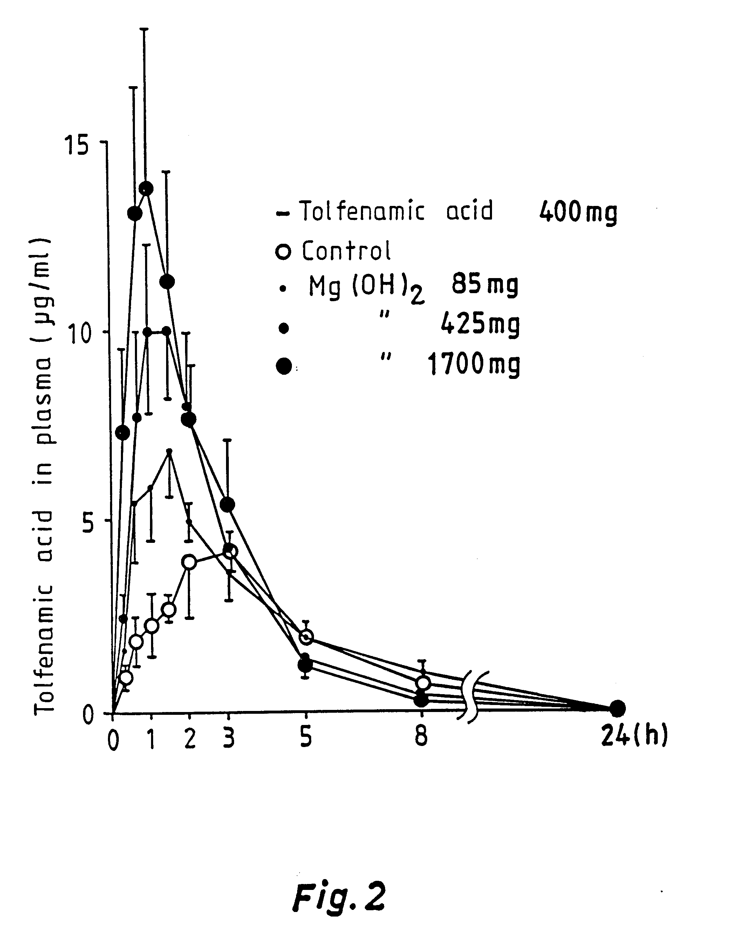

Six test persons were subjected to a cross-over study and orally given magnesium hydroxide (within the range of 85 mg - 1700 mg) and fenamate (tolfenamic acid, 400 mg, 2 tablets "Clotam"(reg.trade mark, AS Gea Farmaceutisk Fabrik, Denmark) 200 mg; The absorption of tolfenamic acid occurred rapidly compared with absorption taking place in identical control conditions (= without magnesium hydroxide). The peak concentrations of tolfenamic acid in blood rise in average 2 -3 times higher and are attained appr. 1 hour earlier than in control conditions (fig. 2).

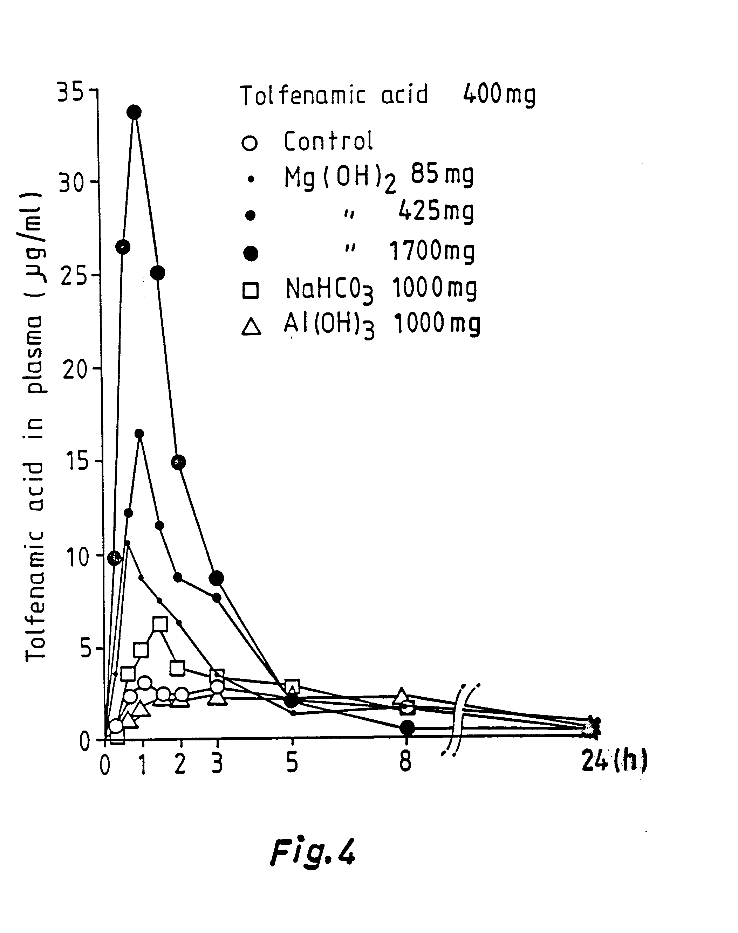

In persons having a poor absorption of fenamates the increase of peak concentrations of e.g. tolfenamic acid can be up to 10-fold through the action of magnesium hydroxide (fig. 4).

The rate of absorption of ibuprofen (400 mg; tabl. "BURANA"; reg.trade mark, Farmos-Yhtymä Oy, Finland) was also accelerated significantly by the concomitant ingestion of Mg-hydroxide.

Since medicines are generally taken with water, the administration of pharmaceutical tablets in the above tests was immediately followed by 150 ml of water with magnesium hydroxide blended therein. With the above dosages, magnesium hydroxide did not cause any side effects.

Thus, magnesium hydroxide is capable of providing an increase in the absorption rate of e.g. tolfenamic acid and ibuprofen.

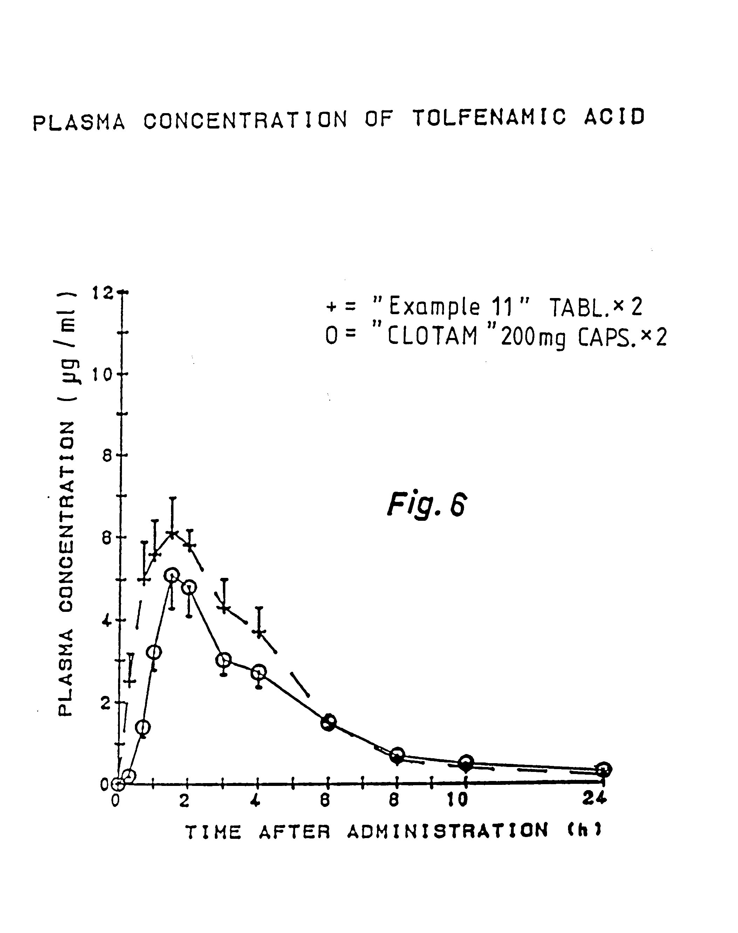

Figure 6 presents the mean results of tests with a pharmaceutical formulation according to the invention containing tolfenamic acid. Said formulation is disclosed in example 11. The results of these tests conducted on twelve volunteeres show the same tendency than those of figures 1 to 5.

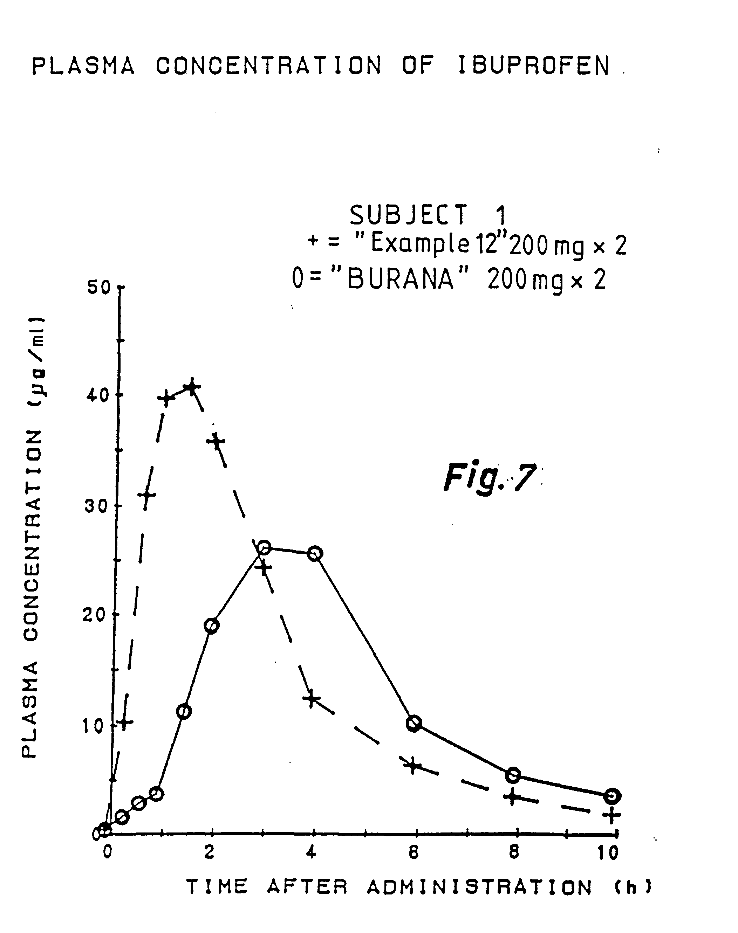

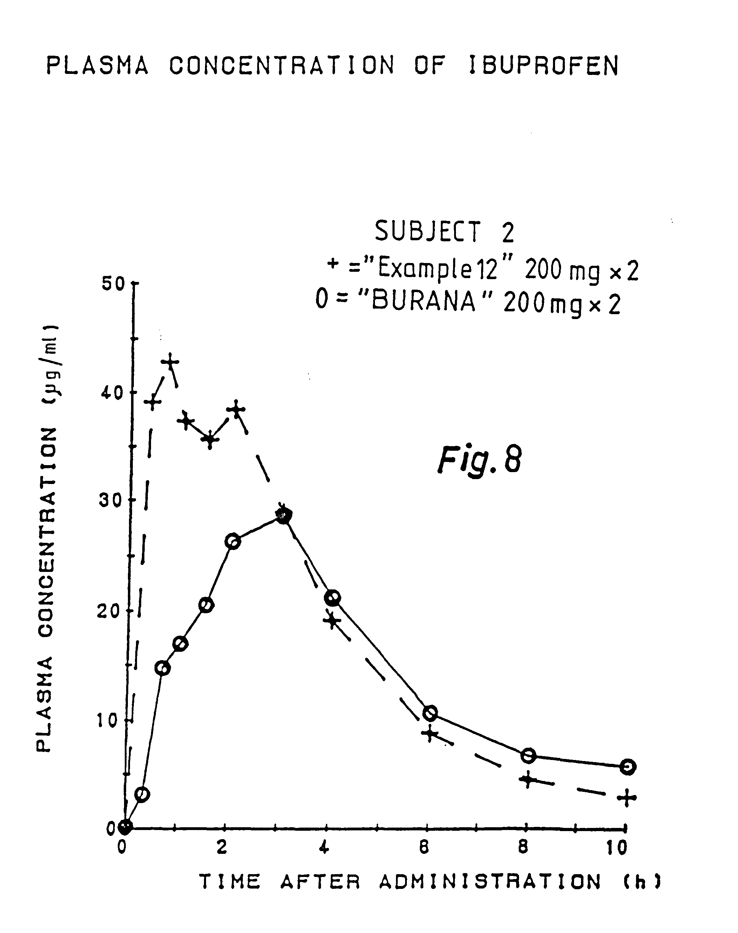

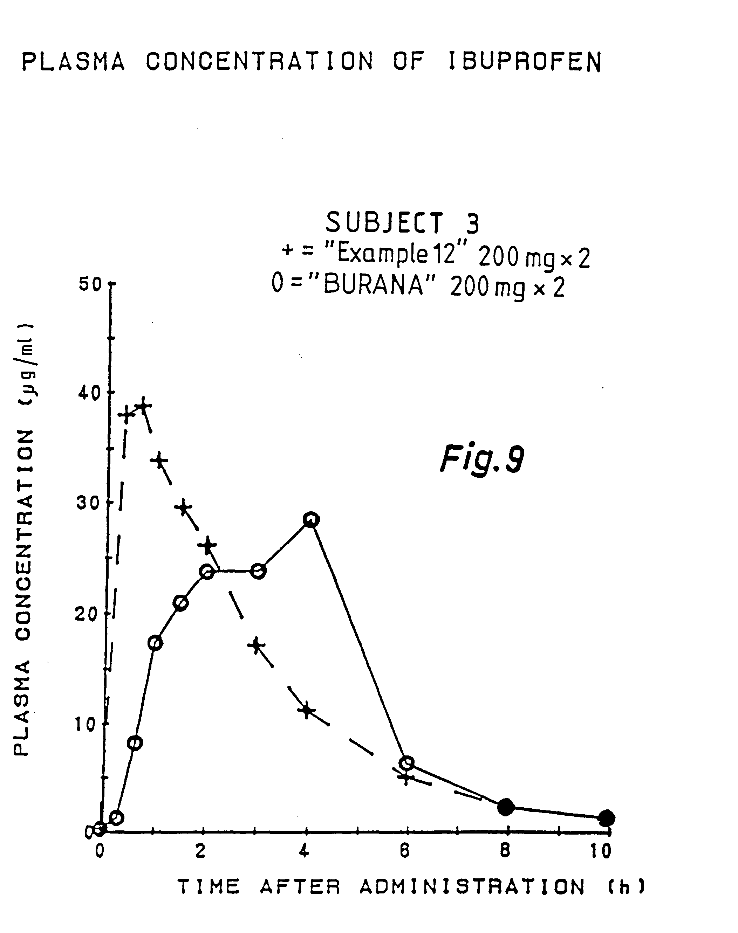

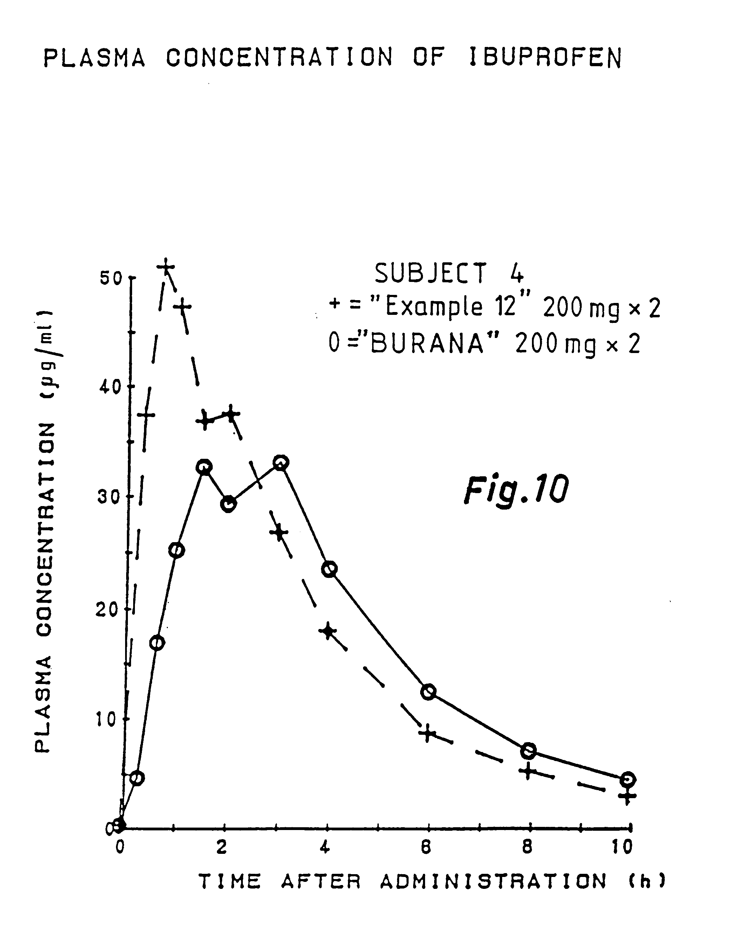

Figures 7-10 reveal the change in plasma concentration after the administration of a formulation according to the invention containing ibuprofen and magnesium oxide (example 12). The figures show the results obtained in four patients. The formulation "Burana", (reg. trade mark, Farmos-Yhtymä Oy, Finland) a conventional ibuprofen formulation, but not containing the alkaline components of this invention, is used as comparison. The results revealed in figures 7 to 10 clearly show that a very rapid and strong rise in the plasma concentration is obtained by the inventive formulation.

Example 1 Tablet formulation

| | |
|---|---|
| 1. Tolfenamic acid | 200 mg |
| 2. Magnesium hydroxide | 200 mg |
| 3. Corn starch | 150 mg |
| 4. Lactose | 100 mg |
| 5. Microcrystalline cellulose | 50 mg |
| 6. Polyvidone (PVP) | 30 mg |
| 7. Magnesium stearate | 5 mg |
| | 735 mg |

The amounts of components 1. - 5. required by a desired production batch are mixed in a suitable dry blender as well as moisturized and granulated with an aqueous or water-in-ethanol solution of component 6. The dried granules are sieved, component 7. added with short mixing and compressing into tablets having a weight of circa 735 mg.

Some of component 3, e.g. 75 mg/tablet, can be added in granules together with component 7.

If desired, the tablets can be coated with a coating film that is rapidly decomposable in stomach.

Example 2 Tablet formulation

The components and preparation process as in example 1 but with the amount of magnesium hydroxide per tablet being 400 mg. The total mass of a tablet will thus be 935 mg and tablets are preferably compressed in the form of capsules.

Example 3 Tablet formulation

The components and preparation process as in example 2 but with the amount of tolfenamic acid per tablet being 100 mg. The total mass of a tablet will thus be 835 mg.

This composition makes it possible to employ a relatively high dosage of magnesium hydroxide compared to the dosage of tolfenamic acid, 400 mg : 100 mg or 800 mg : 200 mg, should the dosage be 2 tablets.

Example 4 Capsule formulation

| 1. Tolfenamic acid | 200 mg |
|---|---|
| 2. Magnesium hydroxide | 200 mg |
| 3. Corn starch | 35 mg |
| 4. Lactose | 50 mg |
| 5. Polyvidone (PVP) | 20 mg |
| 6. Colloidal silica | 10 mg |
| 7. Magnesium stearate | 5 mg |

Components 1 - 4 are mixed with each other in a suitable dry blender and the pulverized mixture is granulated 5. with an aqueous or water-in-ethanol solution. The dried granules are sieved, adding therein a mixture of components 6 and 7 with brief mixing. The finished mixture is metered in covellite capsules of 520 mg each.

Example 5 Capsule formulation

The components and preparation process as in example 4 but with the amount of tolfenamic acid being 100 mg and, respectively, the amount of magnesium hydroxide being 300 mg per tablet. The contents of a capsule have thus a mass of 520 mg.

Example 6 Powdered composition

| 1. Tolfenamic acid | 200 mg |
|---|---|
| 2. Magnesium hydroxide | 200 mg |
| 3. Lactose | 150 mg |

Components 1 - 3 are mixed with each other and metered e.g. in aluminium laminated dosage bags of 550 mg.

For improved handling of the powdered composition, the mixture of components 1 - 3 can be granulated e.g. with polyvidone (25 mg/dose), followed by packing the sieved granules in dosage bags of 575 mg.

Example 7 Powdered composition

| 1. Tolfenamic acid | 200 mg |
|---|---|
| 2. Magnesium hydroxide | 1700 mg |
| 3. Lactose | 300 mg |
| 4. Polyvidone | 80 mg |

Components 1 - 3 are mixed with each other and the powdered mixture is granulated 4. with an aqueous or water-in-ethanol solution. The dried and sieved granules are packed in dosage bags of 2280 mg.

Within the described range of basic composition, the amount of magnesium hydroxide can be varied within the range of 85 - 1700 mg with no trouble.

Example 8 Tablet formulation

The components and preparation process as in example 1 but with lactose replaced by sodium bicarbonate.

Example 9 Capsule formulation

As in example 4 but containing only components 1 and 2.

Example 10 Capsule formulation

As in example 4 but containing only components 1 and 2 and, in addition to those, 100 mg of sodium bicarbonate.

Example 11 Tablet formulation

The tablets are formed following the conventional tablet forming technic of example 1 but using the following composition.

| | |
|---|---|
| Ibuprofen | 200,0 mg |
| Magn.oxid.pond. | 200,0 mg |
| Aerosil 200 | 14,0 mg |
| Ac-Di-Sol | 3,5 mg |
| Mayd. amyl. Rx-1500 | 17,5 mg |
| Carbowax 6000 | 10,5 mg |
| Emcompress | 121,5 mg |
| Amyl.solan | 32,0 mg |
| Avicel PH 105 | 21,0 mg |
| Emcocel 90 M | 45,0 mg |
| Talc. | 31,5 mg |
| Calc. stear | 3,5 mg |
| Spir.fort. | q.s. |
| Aq. purif. | q.s. |
| | 700 mg |

Example 12 Tablet formulation

The preparation process is a conventional one substantially according to example 1 but with the following components:

| | | |
|---|---|---|
| Nucleus: | Tolfenamic acid | 200 mg |
| | Magn.hydroxid.pond. | 200 mg |
| | Cellulos.microcrist. | 140 mg |
| | Polyvidon. 25000 | 30 mg |
| | Natr.stearylfumar | 3 mg |
| Core: | Eudragit$^R$E | 10 mg |
| | Talc. | 2 mg |
| Volatile comp.: | Spir.fort. | q.s. |
| | Isopropanol | q.s. |
| | Aceton | q.s. |

6

EP 0 414 688 B1

In all the above examples, magnesium hydroxide can be replaced by an equivalent weight of magnesium oxide. The exact, stoichiometric equivalence is 40.30 parts of magnesium oxide corresponding to 58,32 mg of magnesium hydroxide.

## Claims

**1.** Formulation with regulated absorption rate comprising a carboxylic acid derivative selected from tolfenamic acid and ibuprofen and an alkaline compound selected from $Mg(OH)_2$, MgO and a mixture of these compounds

**2.** A formulation as set forth in claim 1, **characterized** in that the formulation further comprises conventional formulation components and is in the form of a tablet, capsule, powder, suspension or mixture.

## Patentansprüche

**1.** Stoffmischung mit regulierter Absorptionsrate umfassend ein Carbonsäurederivat, das ausgewählt wird aus einer Tolfenam-Säure und Ibuprofen,
und eine alkalische Verbindung,
die ausgewählt wird aus $Mg(OH)_2$, MgO und einer Mischung aus diesen Verbindungen.

**2.** Stoffmischung nach Anspruch 1, **dadurch gekennzeichnet**, daß die Stoffmischung desweiteren konventionelle Mischungskomponenten umfaßt und die Form einer Tablette, Kapsel, eines Puders, einer Suspension oder einer Mixtur aufweist.

## Revendications

**1.** Formulation présentant une vitesse d'absorption régulée comprenant un dérivé d'un acide carboxylique choisi entre l'acide tolfénamique et l'ibuprofène et un composé alcalin choisi parmi $Mg(OH)_2$, la MgO et un mélange de ces composés.

**2.** Formulation selon la revendication 1, caractérisée en ce que la formulation comprend en outre des composants d'une formulation classique et se présente sous la forme d'un comprimé, d'une capsule, d'une poudre, d'une suspension ou un mélange.

7

*Fig.1*

Fig. 2

Fig.4

PLASMA CONCENTRATION OF TOLFENAMIC ACID

Fig. 6

+ = "Example 11" TABL.×2
O = "CLOTAM" 200mg CAPS.×2

# PLASMA CONCENTRATION OF IBUPROFEN

SUBJECT 1
+ = "Example 12" 200 mg × 2
0 = "BURANA" 200 mg × 2

Fig. 7

PLASMA CONCENTRATION OF IBUPROFEN

SUBJECT 2
+ = "Example 12" 200 mg × 2
0 = "BURANA" 200 mg × 2

Fig. 8

# PLASMA CONCENTRATION OF IBUPROFEN

SUBJECT 3
+ = "Example 12" 200 mg × 2
0 = "BURANA" 200 mg × 2

Fig.9

PLASMA CONCENTRATION OF IBUPROFEN

SUBJECT 4
+ = "Example 12" 200 mg × 2
0 = "BURANA" 200 mg × 2

Fig.10